# EUROPEAN PATENT APPLICATION

(11) **EP 4 427 754 A1**
(43) Date of publication of application: **11.09.2024**
(21) Application number: 22889399.6
(22) Date of filing: 04.11.2022
(51) Int. Cl.: A61K 31/728, A61K 47/36, A61P 37/04, A61P 29/00, A23L 33/125, A61P 1/00

(54) **COMPOSITION CONTAINING HYALURONIC ACIDS OR SALTS THEREOF FOR IMPROVING IMMUNITY OF ORGANISM AND PROMOTING HEALTH OF GASTROINTESTINAL TRACT, AND USE THEREOF**

(30) Priority: 04.11.2021 CN 202111302353
(71) Applicant: Bloomage Biotechnology Corporation Limited, Jinan, Shandong 250101 (CN)
(72) Inventor: SHI, Yanli, Jinan, Shandong 250101 (CN); LIU, Cuihua, Jinan, Shandong 250101 (CN); GUO, Xueping, Jinan, Shandong 250101 (CN); FAN, Shengnan, Jinan, Shandong 250101 (CN); FENG, Ning, Jinan, Shandong 250101 (CN); ZHAO, Lianzhen, Jinan, Shandong 250101 (CN)
(74) Representative: Abel & Imray LLP
(86) International application number: PCT/CN2022/129773
(87) International publication number: WO 2023/078378

(57) **Abstract**

A composition containing hyaluronic acids or salts thereof for improving the immunity of an organism and protecting the health of the gastrointestinal tract, and the use thereof. The composition containing hyaluronic acids or salts thereof contains hyaluronic acids of three molecular weights or salts thereof. The composition achieves the effects of enhancing immunity, alleviating intestinal inflammation, promoting intestinal peristalsis and regulating intestinal flora by means of a synergistic effect; can be added to food and health-care food as a functional ingredient; and can also be used as an excipient in combination with a drug.

## Description

### TECHNICAL FIELD

The present application relates to the technical fields of food, health-care product or pharmaceutical, and in particular to a composition containing hyaluronic acids or salts thereof for improving organism immunity and promoting gastrointestinal health and use thereof.

### BACKGROUND ART

Hyaluronic acid (HA) is an acidic mucopolysaccharide composed of repeated linkage of disaccharide units of D-glucuronic acid and N-acetylglucosamine. It is widely present in the intercellular matrix of animal tissues and in the capsule of certain bacteria. At present, hyaluronic acid has been widely used in cosmetics, pharmaceutical and other fields. With the in-depth research and understanding of hyaluronic acid both domestically and internationally, its application in the food field, especially in functional foods, has also shown broad and unique value.

The sudden outbreak of COVID-19 has spread across the world, and most countries have not yet achieved effective control over the epidemic. Under such circumstances, strengthening normalized prevention and control and improving organism autoimmunity have become the keys to preventing COVID-19 and resisting the disease. Immunity is the body's own defense mechanism. It is the ability for human body to identify and eliminate any foreign matter (virus, bacteria, etc.) that invades from the outside, to deal with damaged, aging, dead, and denatured own cells, and to identify and deal with mutant cells and virus-infected cells in the body. Improving immunity is the physiological response of the human body to identify and eliminate "dissidents", which can be enhanced in certain ways. In addition to exercise, it is also recommended to eat more beneficial foods, especially functional foods that can regulate immune factors and improve resistance.

The gastrointestinal tract is the largest immune and detoxification organ of the human body and has a decisive impact on health. More than 70% of the immune cells in the human body are located on the gastrointestinal mucosa, forming an immune barrier in the intestine to resist bacteria and viruses, and maintain the stability of the intestinal environment. In recent years, however, the morbidity of gastrointestinal diseases, such as gastrointestinal inflammation, gastrointestinal stones, gastric ulcers, intestinal dysbacteriosis, etc. has become higher and higher. More and more people are suffering from intestinal diseases. Therefore, the development of raw materials and products that promote intestinal health, which can prevent and relieve intestinal inflammation, promote intestinal peristalsis, and regulate intestinal flora, can meet the needs of the market and people, and also has considerable economic benefits.

At present, research on HA is becoming increasingly in-depth, and as a functional raw material, HA has a certain effect on improving human immunity. Chinese patent application CN1498626A entitled "Application of hyaluronic acid in improving human immunity and improving symptoms of immune deficiency" discloses a composition containing hyaluronic acid as an active ingredient. The product has no toxic effect and has a function of improving immunity in both mouse and human experiments. However, the specific molecular weight of hyaluronic acid is not disclosed, which fails to reveal the function of hyaluronic acid with different molecular weights, providing a low reference value. Chinese patent CN108498549B entitled "Composition for improving immune function and the application thereof' discloses a mixture of hyaluronic acid and lactic acid bacteria for alleviating and treating complications induced by anti-tumor chemotherapy, which reduces chemotherapy drug side effects, and improves immune deficiency. The method requires interaction with lactic acid bacteria which need to be prepared into freeze-dried bacterial powder. In addition, the composition also requires the addition of other excipients, such as freeze-dried protectants and forming agents. The operation is complex, with a variety of added substances, and the effectiveness of hyaluronic acid with different molecular weights has not been fully exerted.

In addition, HA also has certain effects in promoting gastrointestinal health. Research from the University of Oklahoma Health Science Center shows that 35 kDa HA can enhance epithelial barrier function and prevent the occurrence of necrotizing enterocolitis in mice. There are also relevant domestic literature reports that treatment with 530 kDa hyaluronic acid can play a protective role against mouse intestinal infections caused by pathogenic bacteria (Listeria monocytogenes, Citrobacter bacteria, and enteropathogenic *Escherichia coli*). These experiments used a single molecular weight of HA, which had a single effect.

Therefore, in order to solve the problem of the single efficacy of single molecular weight of HA, the present application intends to provide a HA complex and a product thereof with clear ingredients and significant effects, so as to give full play to the role of hyaluronic acid in improving immunity and protecting gastrointestinal health.

### CONTENTS OF THE APPLICATION

In view of the above problems, the application provides a composition of hyaluronic acids or salts thereof for improving organism immunity and promoting gastrointestinal health. The composition takes hyaluronic acids or salts thereof with different molecular weights as components. According to the different functions of hyaluronic acid with different molecular weights, regulation for the immunity, improvement for the ability to resist gastrointestinal inflammation, as well as promotion for the intestinal peristalsis and the growth of intestinal probiotics can be achieved through synergistic effects.

The specific technical solutions for the present application are as follows:
1. A composition containing hyaluronic acids or salts thereof, which comprises hyaluronic acids or salts thereof with three different molecular weights, wherein:
   the molecular weight of the hyaluronic acid or a salt thereof with a first molecular weight is between 10 kDa and 200 kDa, preferably between 10 kDa and 100 kDa, further preferably between 30 kDa and 50 kDa;
   the molecular weight of the hyaluronic acid or a salt thereof with a second molecular weight is between 200 kDa and1000kDa, preferably between 200 kDa and600 kDa, further preferably between 200 kDa and 400kDa;
   the molecular weight of the hyaluronic acid or a salt thereof with a third molecular weight is between 1000 kDa and 1500 kDa, preferably between 1300 kDa and 1500 kDa.
2. The composition according to item 1, wherein based on a total part of 10 parts by weight after mixing the three different hyaluronic acids
   the hyaluronic acid or a salt thereof with the first molecular weight is 1-4 parts, preferably 2-4 parts;
   the hyaluronic acid or a salt thereof with the second molecular weight is 3-6 parts, preferably 4-6 parts; and
   the hyaluronic acid or a salt thereof with the third molecular weight is 1-3 parts, .
3. The composition according to any one of items 1-2, wherein the hyaluronic acid or salt thereof is one or two or more selected from the group consisting of hyaluronic acid, sodium hyaluronate, potassium hyaluronate, calcium hyaluronate and zinc hyaluronate, preferably sodium hyaluronate or potassium hyaluronate.
4. Use of the composition according to any one of items 1-3 in the fields of food, health-care product or pharmaceutical.
5. A food composition comprising the composition according to any one of items 1-3.
6. The food composition according to item 5, wherein the food composition is used to improve organism immunity, relieve intestinal inflammation, increase intestinal peristalsis and/or regulate intestinal flora.
7. A health-care product composition comprising the composition according to any one of items 1-3.
8. The health-care product composition according to item 7, wherein the health-care product composition is used to improve organism immunity, relieve intestinal inflammation, increase intestinal peristalsis and/or regulate intestinal flora.
9. A pharmaceutical composition comprising the composition according to any one of items 1-3.
10. The pharmaceutical composition according to item 9, wherein the pharmaceutical composition is used to improve organism immunity, relieve intestinal inflammation, increase intestinal peristalsis and/or regulate intestinal flora.
11. A method of improving organism immunity, relieving intestinal inflammation, increasing intestinal peristalsis and/or regulating intestinal flora, which comprises administering the composition of any one of items 1-3 to a subject.
12. Use of the composition according to any one of items 1-3 for improving organism immunity, relieving intestinal inflammation, increasing intestinal peristalsis and/or regulating intestinal flora.
13. Use of the composition of any one of items 1-3 in the preparation of a medicament for improving organism immunity, relieving intestinal inflammation, increasing intestinal peristalsis and/or regulating intestinal flora.

### Effect of invention

1. The composition provided by the present application has the advantage in term of clear ingredients, simple operation, and convenient for preparation and use.
2. The composition provided by the present application has significant effects on improving organism immunity, relieving gastrointestinal inflammation, increasing intestinal peristalsis, increasing growth of intestinal probiotics, and protecting the gastric mucosa, etc. It may be added as a functional ingredient to food and health-care food. It may also be used as an excipient and used in combination with a drug.

### DETAILED DESCRIPTION

This application will be described in detail below. Although specific embodiments of the present application are shown, it should be understood that the present application may be implemented in various forms and should not be limited to the embodiments set forth herein. Rather, these embodiments are provided to provide for thorough understanding of the present application, and to fully convey the scope of the present application to those skilled in the art.

It should be noted that certain words are used in the description and claims to refer to specific components. Those skilled in the art will understand that skilled persons may use different nouns to refer to the same component. This description and the claims do not use difference in nouns as a way to distinguish components, but rather use differences in functions of the components as a criterion for distinction. If the words "comprise" or "include" mentioned throughout the description and claims are openended terms, they should be interpreted as "including but not limited to." The following descriptions of the description are preferred embodiments for implementing the present application. However, the descriptions are for the purpose of general principles of the description and are not intended to limit the scope of the present application. The scope of protection of this application shall be determined by the appended claims.

The present application provides a composition containing hyaluronic acids or salts thereof, which comprises hyaluronic acids of three different molecular weights or salts thereof,
the molecular weight of the hyaluronic acid or a salt thereof with a first molecular weight is between 10 kDa and 200 kDa, preferably between 10 kDa and 100 kDa, further preferably between 30 kDa and 50 kDa;
the molecular weight of the hyaluronic acid or a salt thereof with a second molecular weight is between 200 kDa and 1000 kDa, preferably between 200 kDa and 600 kDa, further preferably between 200 kDa and 400 kDa; and
the molecular weight of the hyaluronic acid or a salt thereof with a third molecular weight is between 1000 kDa and 1500 kDa, preferably between 1300 kDa and 1500 kDa.

In the present application, the molecular weight refers to a weight average molecular weight.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 100 kDa; the molecular weight of the hyaluronic acid or a salt thereof with a second molecular weight is between 200 kDa and 1000 kDa; and the molecular weight of the hyaluronic acid or a salt thereof with a third molecular weight is between 1000 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and100 kDa; the molecular weight of the hyaluronic acid or a salt thereof with a second molecular weight is between 200 kDa and 600 kDa; and the molecular weight of the hyaluronic acid or a salt thereof with a third molecular weight is between 1300 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kD and -100 kDa; the molecular weight of the hyaluronic acid or a salt thereof with a second molecular weight is between 200 kDa-600 kDa; and the molecular weight of the hyaluronic acid or a salt thereof with a third molecular weight is between 1000 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 100 kDa; the molecular weight of the hyaluronic acid or a salt thereof with a second molecular weight is between 200 kDa and 400 kDa; and the molecular weight of the hyaluronic acid or a salt thereof with a third molecular weight is between 1000 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 100 kDa; the molecular weight of the hyaluronic acid or a salt thereof with a second molecular weight is between 200 kD and -400 kDa; and the molecular weight of the hyaluronic acid or a salt thereof with a third molecular weight is between 1300 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 100 kDa; the molecular weight of the hyaluronic acid or a salt thereof with a second molecular weight is between 200 kDa and1000 kDa; and the molecular weight of the hyaluronic acid or a salt thereof with a third molecular weight is between 1300 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 200 kDa; the molecular weight of the hyaluronic acid or a salt thereof with a second molecular weight is between 200 kDa and 600 kDa; and the molecular weight of the hyaluronic acid or a salt thereof with a third molecular weight is between 1300 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 200 kDa; the molecular weight of the hyaluronic acid or a salt thereof with a second molecular weight is between 200 kDa and 600 kDa; and the molecular weight of the hyaluronic acid or a salt thereof with a third molecular weight is between 1000 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 00 kDa; the molecular weight of the hyaluronic acid or a salt thereof with a second molecular weight is between 200 kDa and 1000 kDa; and the molecular weight of the hyaluronic acid or a salt thereof with a third molecular weight is between 1000 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 200 kDa; the molecular weight of the hyaluronic acid or a salt thereof with a second molecular weight is between 200 kDa and 1000 kDa; and the molecular weight of the hyaluronic acid or a salt thereof with a third molecular weight is between 1300 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 200 kDa; the molecular weight of the hyaluronic acid or a salt thereof with a second molecular weight is between 200 kDa and 400 kDa; and the molecular weight of the hyaluronic acid or a salt thereof with a third molecular weight is between 1000 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 200 kDa; the molecular weight of the hyaluronic acid or a salt thereof with a second molecular weight is between 200 kDa and 400 kDa; and the molecular weight of the hyaluronic acid or a salt thereof with a third molecular weight is between 1300 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 200 kDa and 1000 kDa; and the molecular weight of the hyaluronic acid or a salt thereof with a third molecular weight is between 1000 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 200 kDa and 1000 kDa; and the molecular weight of the hyaluronic acid or a salt thereof with a third molecular weight is between 1300 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 20 kDa and 600 kDa; and the molecular weight of the hyaluronic acid or a salt thereof with a third molecular weight is between 1000 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 20 kDa and 600 kDa; and the molecular weight of the hyaluronic acid or a salt thereof with a third molecular weight is between 1300 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 20 kDa and 400 kDa; and the molecular weight of the hyaluronic acid or a salt thereof with a third molecular weight is between 1000 kDa and 1500 kDa.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 20 kDa and 400 kDa; and the molecular weight of the hyaluronic acid or a salt thereof with a third molecular weight is between 1300 kDa and 1500 kDa.

For Example, the molecular weight of the hyaluronic acid or a salt thereof with the first molecular weight may be 10 kDa, 20 kDa, 30 kDa, 40 kDa, 50 kDa, 60 kDa, 70 kDa, 80 kDa, 90 kDa, 100 kDa, 160 kDa, 200 kDa, etc.;
the molecular weight of the hyaluronic acid or a salt thereof with the second molecular weight may be 200 kDa, 280 kD, 300 kDa, 400 kDa, 500 kDa, 600 kDa, 700 kDa, 800 kDa, 900 kDa, 1000 kDa, etc.;
the molecular weight of the hyaluronic acid or a salt thereof with the third molecular weight may be 1000 kDa, 1100 kDa, 1200 kDa, 1300 kDa, 1350 kDa, 1400 kDa, 1500 kDa, etc..

In one embodiment, wherein based on a total part of 10 parts by weight after mixing the three different hyaluronic acids,
the hyaluronic acid or a salt thereof with a first molecular weight is 1-4 parts, preferably 2-4 parts;
the hyaluronic acid or a salt thereof with a second molecular weight is 3-6 parts, preferably 4-6 parts; and
the hyaluronic acid or a salt thereof with a third molecular weight is 1-3 parts.

For example, counted by parts by weight, the total part after mixing is 10 parts,
the hyaluronic acid or a salt thereof with the first molecular weight may be 1 part, 2 parts, 3 parts, 4 parts, etc.;
the hyaluronic acid or a salt thereof with the second molecular weight may be 3 parts, 4 parts, 5 parts, 6 parts, etc.;
the hyaluronic acid or a salt thereof with the third molecular weight may be 1 part, 2 parts, 3 parts, etc.

In the above specific embodiments, for example, 1-4 parts, preferably 2-4 parts, of the hyaluronic acid or a salt thereof with the first molecular weight; 3-6 parts, preferably 4-6 parts of the hyaluronic acid or a salt thereof with the second molecular weight; and 1-3 parts of the hyaluronic acid or a salt thereof with the third molecular weight are weighed and mixed to obtain the composition containing hyaluronic acids or salts thereof described herein.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 100 kDa; the molecular weight of the hyaluronic acid or a salt thereof with a second molecular weight is between 200 kDa and 400 kDa; and the molecular weight of the hyaluronic acid or a salt thereof with a third molecular weight is between 1000 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 100 kDa; the molecular weight of the hyaluronic acid or a salt thereof with a second molecular weight is between 200 kDa and 400 kDa; and the molecular weight of the hyaluronic acid or a salt thereof with a third molecular weight is between 1300 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 100 kDa; the molecular weight of the hyaluronic acid or a salt thereof with a second molecular weight is between 200 kDa and 600 kDa; and the molecular weight of the hyaluronic acid or a salt thereof with a third molecular weight is between 1000 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 100 kDa; the molecular weight of the hyaluronic acid or a salt thereof with a second molecular weight is between 200 kDa and 400 kDa; and the molecular weight of the hyaluronic acid or a salt thereof with a third molecular weight is between 1000 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 100 kDa; the molecular weight of the hyaluronic acid or a salt thereof with a second molecular weight is between 200 kDa and 400 kDa; and the molecular weight of the hyaluronic acid or a salt thereof with a third molecular weight is between 1300 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 100 kDa; the molecular weight of the hyaluronic acid or a salt thereof with a second molecular weight is between 200 kDa and 1000 kDa; and the molecular weight of the hyaluronic acid or a salt thereof with a third molecular weight is between 1300 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 200 kDa; the molecular weight of the hyaluronic acid or a salt thereof with a second molecular weight is between 200 kDa and 600 kDa; and the molecular weight of the hyaluronic acid or a salt thereof with a third molecular weight is between 1300 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 200 kDa; the molecular weight of the hyaluronic acid or a salt thereof with a second molecular weight is between 200 kDa and 600 kDa; and the molecular weight of the hyaluronic acid or a salt thereof with a third molecular weight is between 1000 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 200 kDa; the molecular weight of the hyaluronic acid or a salt thereof with a second molecular weight is between 200 kDa and 1000 kDa; and the molecular weight of the hyaluronic acid or a salt thereof with a third molecular weight is between 1000 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 200 kDa; the molecular weight of the hyaluronic acid or a salt thereof with a second molecular weight is between 200 kDa and 1000 kDa; and the molecular weight of the hyaluronic acid or a salt thereof with a third molecular weight is between 1300 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 200 kDa; the molecular weight of the hyaluronic acid or a salt thereof with a second molecular weight is between 200 kDa and 400 kDa; and the molecular weight of the hyaluronic acid or a salt thereof with a third molecular weight is between 1000 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 10 kDa and 200 kDa; the molecular weight of the hyaluronic acid or a salt thereof with a second molecular weight is between 200 kDa and 400 kDa; and the molecular weight of the hyaluronic acid or a salt thereof with a third molecular weight is between 1300 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 200 kDa and 1000 kDa; and the molecular weight of the hyaluronic acid or a salt thereof with a third molecular weight is between 1000 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 20 kDa and 1000 kDa; and the molecular weight of the hyaluronic acid or a salt thereof with a third molecular weight is 1300 kDa-1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 20 kDa and 600 kDa; and the molecular weight of the hyaluronic acid or a salt thereof with a third molecular weight is between 1000 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 20 kDa and 600 kDa; and the molecular weight of the hyaluronic acid or a salt thereof with a third molecular weight is between 1300 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 20 kDa and 400 kDa; and the molecular weight of the hyaluronic acid or a salt thereof with a third molecular weight is between 1000 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the present application provides a composition containing hyaluronic acids or salts thereof, which comprises hyaluronic acids of three different molecular weights or salts thereof, wherein the molecular weight of the hyaluronic acid or a salt thereof with a first molecular weight is between 30 kDa and 50 kDa; the molecular weight of the hyaluronic acid or a salt thereof with a second molecular weight is between 200 kDa and 400 kDa; and the molecular weight of the hyaluronic acid or a salt thereof with a third molecular weight is between 1300 kDa and 1500 kDa; based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

In one embodiment, the hyaluronic acid or salt thereof is one or two or more selected from the group consisting of hyaluronic acid, sodium hyaluronate, potassium hyaluronate, calcium hyaluronate and zinc hyaluronate, preferably sodium hyaluronate or potassium hyaluronate.

The application provides the use of the composition described above in the fields of food, health-care product or pharmaceutical.

By applying the composition described above to the fields of food, health-care product or pharmaceutical, the organism immunity may be improved, the gastrointestinal inflammation may be relieved, the intestinal peristalsis and intestinal probiotics growth may be promoted.

The present application provides a food composition comprising the composition described above.

In one embodiment, the food composition may further comprise excipients, which may be, for example, preservatives, food colorings, flavoring agents, emulsifiers, spices, etc.

The present application provides a health-care product composition comprising the composition described above.

In one embodiment, the health-care product composition may further comprise excipients, such as preservatives, food colorings, flavoring agents, emulsifiers, spices, etc.

The present application provides a pharmaceutical composition comprising the composition described above.

In one embodiment, the pharmaceutical composition may further comprise excipients, which may be, for example, lubricants, adhesives, colorants, diluents, absorbents, etc.

In the present application, the excipients refer to compositions or composition components that are generally non-toxic, inert and/or physiologically compatible.

The dosage forms of the food compositions, health-care product compositions, and pharmaceutical compositions described above are not limited, and may be, for example, solid form, semi-solid form, or liquid form, etc.

The present application provides a method for improving organism immunity, relieving intestinal inflammation, increasing intestinal peristalsis and/or regulating intestinal flora, which comprises administering the composition described above to a subj ect.

In the present application, there is no limitation on the method of administration. For Example, the method of administration may be oral administration.

In the present application, the subject means to any animal (e.g., mammal), including but not limited to: humans, non-human primates, canines, felines, rodents, and the like.

The present application provides use of the composition described above for improving organism immunity, relieving intestinal inflammation, increasing intestinal peristalsis and/or regulating intestinal flora.

The present application provides use of the composition described above in the preparation of a medicament for improving organism immunity, relieving intestinal inflammation, increasing intestinal peristalsis and/or regulating intestinal flora.

### Example

The present application provides a general and/or specific description of the materials and test methods used in the experiment. In the following examples, if there are no other special instructions, % means wt%, that is, weight percentage. If the manufacturer of the reagents or instruments used is not indicated, they are all conventional reagent products that can be purchased commercially. Among them, hyaluronic acid or salt thereof with the first molecular weight, hyaluronic acid or salt thereof with the second molecular weight, and hyaluronic acid or salt thereof with the third molecular weight were provided by BLOOMAGE BIOTECHNOLOGY

### CORPORATION LIMITED.

### Example 1

The first molecular weight of sodium hyaluronate with a molecular weight of 40 kDa, the second molecular weight of sodium hyaluronate with a molecular weight of 280 kDa, and the third molecular weight of sodium hyaluronate with a molecular weight of 1350 kDa were mixed in a mass ratio of 3:5:2, mixing thoroughly for later use.

### Example 2

The first molecular weight of sodium hyaluronate with a molecular weight of 40 kDa, the second molecular weight of sodium hyaluronate with a molecular weight of 600 kDa, and the third molecular weight of sodium hyaluronate with a molecular weight of 1350 kDa were mixed in a mass ratio of 3:5:2, mixing thoroughly for later use.

### Example 3

The first molecular weight of sodium hyaluronate with a molecular weight of 40 kDa, the second molecular weight of sodium hyaluronate with a molecular weight of 280 kDa, and the third molecular weight of sodium hyaluronate with a molecular weight of 1000 kDa were mixed in a mass ratio of 3:5:2, mixing thoroughly for later use.

### Example 4

The first molecular weight of sodium hyaluronate with a molecular weight of 160 kDa, the second molecular weight of sodium hyaluronate with a molecular weight of 280 kDa, and the third molecular weight of sodium hyaluronate with a molecular weight of 1350 kDa were mixed in a mass ratio of 3:5:2, mixing thoroughly for later use.

### Example 5

The first molecular weight of sodium hyaluronate with a molecular weight of 160 kDa, the second molecular weight of sodium hyaluronate with a molecular weight of 600 kDa, and the third molecular weight of sodium hyaluronate with a molecular weight of 1350 kDa were mixed in a mass ratio of 3:5:2, mixing thoroughly for later use.

### Example 6

The first molecular weight of sodium hyaluronate with a molecular weight of 160 kDa, the second molecular weight of sodium hyaluronate with a molecular weight of 280 kDa, and the third molecular weight of sodium hyaluronate with a molecular weight of 1000 kDa were mixed in a mass ratio of 3:5:2, mixing thoroughly for later use.

### Example 7

The first molecular weight of sodium hyaluronate with a molecular weight of 40 kDa, the second molecular weight of sodium hyaluronate with a molecular weight of 600 kDa, and the third molecular weight of sodium hyaluronate with a molecular weight of 1000 kDa were mixed in a mass ratio of 3:5:2, mixing thoroughly for later use.

### Example 8

The first molecular weight of sodium hyaluronate with a molecular weight of 160 kDa, the second molecular weight of sodium hyaluronate with a molecular weight of 600 kDa, and the third molecular weight of sodium hyaluronate with a molecular weight of 1000 kDa were mixed in a mass ratio of 3:5:2, mixing thoroughly for later use.

### Example 9

The first molecular weight of sodium hyaluronate with a molecular weight of 40 kDa, the second molecular weight of sodium hyaluronate with a molecular weight of 280 kDa, and the third molecular weight of sodium hyaluronate with a molecular weight of 1350 kDa were mixed in a mass ratio of 3:6: 1, mixing thoroughly for later use.

### Example 10

The first molecular weight of sodium hyaluronate with a molecular weight of 40 kDa, the second molecular weight of sodium hyaluronate with a molecular weight of 280 kDa, and the third molecular weight of sodium hyaluronate with a molecular weight of 1350 kDa were mixed in a mass ratio of 2:5:3, mixing thoroughly for later use.

### Example 11

The first molecular weight of sodium hyaluronate with a molecular weight of 40 kDa, the second molecular weight of sodium hyaluronate with a molecular weight of 280 kDa, and the third molecular weight of sodium hyaluronate with a molecular weight of 1350 kDa were mixed in a mass ratio of 1:3:1, mixing thoroughly for later use.

### Comparative Example 1

The first molecular weight of sodium hyaluronate with a molecular weight of 40 kDa was dissolved in physiological saline according to the concentration required for each experiment, completely dissolving for later use.

### Comparative Example 2

The third molecular weight of sodium hyaluronate with a molecular weight of 1350 kDa was dissolved in physiological saline according to the concentration required for each experiment, completely dissolving for later use.

### Comparative Example 3

The first molecular weight of sodium hyaluronate with a molecular weight of 40 kDa and the third molecular weight of sodium hyaluronate with a molecular weight of 1350 kDa were mixed in a mass ratio of 3:2, mixing thoroughly for later use.

### Comparative Example 4

The second molecular weight of sodium hyaluronate with a molecular weight of 280 kDa was dissolved in physiological saline according to the concentration required for each experiment, completely dissolving for later use.

### Comparative Example 5

The second molecular weight of sodium hyaluronate with a molecular weight of 280 kDa and the third molecular weight of sodium hyaluronate with a molecular weight of 1350 kDa were mixed in a mass ratio of 5:2, mixing thoroughly for later use.

### Comparative Example 6

The sodium hyaluronate with a molecular weight of 80 kDa was dissolved in physiological saline according to the concentration required for each experiment, completely dissolving for later use.

### Comparative Example 7

The first molecular weight of sodium hyaluronate with a molecular weight of 40 kDa and the second molecular weight of sodium hyaluronate with a molecular weight of 280 kDa were mixed in a mass ratio of 3:5, mixing thoroughly for later use.

**Table 1 Usage table of components used in Examples and Comparative**

| Examples | | | | |
|---|---|---|---|---|
| | The molecular weight of hyaluronate of the first molecular weight | The molecular weight of hyaluronate of the second molecular weight | The molecular weight of hyaluronate of the third molecular weight | The mass ratio |
| Example 1 | 40kDa | 280kDa | 1350kDa | 3: 5: 2 |
| Example 2 | 40kDa | 600kDa | 1350kDa | 3: 5: 2 |
| Example 3 | 40kDa | 280kDa | 1000kDa | 3: 5: 2 |
| Example 4 | 160kDa | 280kDa | 1350kDa | 3: 5: 2 |
| Example 5 | 160kDa | 600kDa | 1350kDa | 3: 5: 2 |
| Example 6 | 160kDa | 280kDa | 1000kDa | 3: 5: 2 |
| Example 7 | 40kDa | 600kDa | 1000kDa | 3: 5: 2 |
| Example 8 | 160kDa | 600kDa | 1000kDa | 3: 5: 2 |
| Example 9 | 40kDa | 280kDa | 1350kDa | 3: 6: 1 |
| Example 10 | 40kDa | 280kDa | 1350kDa | 2: 5: 3 |
| Example 11 | 40kDa | 280kDa | 1350kDa | 1: 3: 1 |
| Comparative Example 1 | 40kDa | - | - | - |
| Comparative Example 2 | - | - | 1350kDa | - |
| Comparative Example 3 | 40kDa | - | 1350kDa | 3: 2 |
| Comparative Example 4 | - | 280kDa | - | - |
| Comparative Example 5 | - | 280kDa | 1350kDa | 5: 2 |
| Comparative Example 6 | 8kDa | - | - | - |
| Comparative Example 7 | 40kDa | 280kDa | - | 3: 5 |

### Experimental Materials

Different strains of zebrafish used in the experiment were provided by the Zebrafish Drug Screening Platform of Biology Institute of Shandong Academy of Sciences; hyaluronic acid samples were provided by BLOOMAGE

### BIOTECHNOLOGY CORPORATION LIMITED.

### Instruments and equipment

SZX16 fluorescence microscope and DP2-BSW image acquisition system were purchased from Japanese Olympus Company; Forma 3111 water-jacketed CO2 incubator was purchased from American Forma Company); zebrafish breeding and feeding equipment was purchased from Beijing Aisheng Biotechnology Co., Ltd.

### Experimental Example 1 Experiment on regulating immunity

Using the zebrafish strain Tg (lyz: GFP) as the model, after fertilized eggs developed for 48 hpf (hour post fertilization), an immunosuppressive model was created by the treatment with the immunosuppressant chloramphenicol, and hyaluronic acid samples were added for processing. After sample processing for 24 hours, the sample was observed under a fluorescence microscope, taking pictures, counting the number of immune cells, and using GraphPad software to perform statistical analysis on the data. The results are shown in Table 2.

**Table 2 Effects of hyaluronic acid composition on zebrafish immune cells**

| Group | Dosage(µg/mL) | The number of the immune cells |
|---|---|---|
| Control group | 200 | 55.4±4.06 |
| Model group | 200 | 29.4±3.68^{##} |
| Example 1 | 200 | 54.2±6.31** |
| Example 2 | 200 | 49.0±3.09** |
| Example 3 | 200 | 46.4±4.76** |
| Example 4 | 200 | 48.5±1.07** |
| Example 5 | 200 | 47.3±2.68** |
| Example 6 | 200 | 45.2±2.02** |
| Example 7 | 200 | 45.9±5.18** |
| Example 8 | 200 | 44.0±6.00** |
| Example 9 | 200 | 50.2±1.80** |
| Example 10 | 200 | 53.1±3.09** |
| Example 11 | 200 | 51.7±2.24** |
| Comparative Example 1 | 200 | 37.6±1.86 |
| Comparative Example 2 | 200 | 40.5±3.07* |
| Comparative Example 3 | 200 | 42.0±1.89* |
| Comparative Example 4 | 200 | 36.2±4.03 |
| Comparative Example 5 | 200 | 41.3±2.97* |
| Comparative Example 6 | 200 | 32.3±2.11 |
| Comparative Example 7 | 200 | 40.9±3.04 |

| | | |
|---|---|---|
| Note: Compared with the control group, #: P<0.05, significant; ##: P<0.01, extremely significant. Compared with the model group, *: P<0.05, significant; **: P<0.01, extremely significant. | | |

As can be seen from Table 2, compared with the control group, the immune cells in the model group were disordered and the number was significantly reduced, indicating that the model was successfully constructed; compared with the model group, the number of zebrafish immune cells in the sample group was significantly increased, especially, the effect in Example 1 is the best, followed by Examples 10, 11, 9, and 2, indicating that feeding zebrafish a hyaluronic acid composition will significantly improve its immune activity.

### Experimental Example 2 Experiment on regulating intestinal peristalsis activity

Wild AB strain zebrafish with development of 5dpf (day post fertilization) were used as experimental animals to study the effect of hyaluronic acid samples on intestinal peristalsis. After pre-staining the zebrafish intestine with calcein, the mean fluorescence intensity (IOD) value of the intestine was calculated. The sample was then incubated with the pre-stained zebrafish in the dark, and the IOD of the zebrafish intestine after sample treatment was calculated. According to the formula: intestinal IOD coefficient = (mean intestinal IOD value before sample treatment - mean intestinal IOD value after sample treatment) /mean intestinal IOD value before sample treatment, the intestinal IOD coefficient of zebrafish in the sample treatment group was calculated, and the statistical analysis was performed. The results are shown in Table 3.

**Table 3 Experiment on the effect of hyaluronic acid composition on zebrafish intestinal peristalsis.**

| Group | Dosage (µg/mL) | Intestinal IOD coefficient |
|---|---|---|
| The control group | 200 | 0.41±0.024 |
| Example 1 | 200 | 0.87±0.015** |
| Example 2 | 200 | 0.77±0.034** |
| Example 3 | 200 | 0.76±0.036** |
| Example 4 | 200 | 0.75±0.021** |
| Example 5 | 200 | 0.70±0.022* |
| Example 6 | 200 | 0.65±0.039* |
| Example 7 | 200 | 0.70±0.060* |
| Example 8 | 200 | 0.62±0.073* |
| Example 9 | 200 | 0.80±0.053** |
| Example 10 | 200 | 0.84±0.044** |
| Example 11 | 200 | 0.82±0.046** |
| Comparative Example 1 | 200 | 0.52±0.013 |
| Comparative Example 2 | 200 | 0.52±0.032 |
| Comparative Example 3 | 200 | 0.56±0.017 |
| Comparative Example 4 | 200 | 0.49±0.042 |
| Comparative Example 5 | 200 | 0.54±0.032 |
| Comparative Example 6 | 200 | 0.43±0.016 |
| Comparative Example 7 | 200 | 0.53±0.022 |

| | | |
|---|---|---|
| Note: Compared with the control group, *: P<0.05, significant; **: P<0.01, extremely significant. | | |

It can be seen from Table 3 that compared with the blank control group, the fluorescent dye in the zebrafish intestine of the sample group was significantly reduced, especially, Example 1 had the best effect, followed by Examples 1 0, 2, 9, and 11, indicating that the hyaluronic acid composition may promote intestinal peristalsis.

### Experimental Example 3 Experiment on promoting the growth of intestinal probiotics

*Lactobacillus rhamnosus, Lactobacillus helveticus,* etc. from the intestinal probiotic group Lactobacillus genus were selected as starting strains. Lactobacillus conventional medium MRS was used as the basic medium. The hyaluronic acids or salts thereof sample accounted for 0.2% (w/v) of the medium volume, the negative control was sugar-free MRS medium, and the positive control was glucose MRS medium with the same concentration as HA. The bacterial concentration (A₆₀₀) at different culture times was detected, and a total of 8 intestinal probiotic strains were selected for proliferation experiments *in vitro.* The results are shown in Table 4.

**Table 4 Effect of hyaluronic acid composition on probiotic growth (OD₆₀₀)**

| Group | *lactob αcillus rham nosus* | *lactoba cillus helveti cus* | *Lactob αcillus fermen tum* | *Lactob αcillus brevis* | *Lactob αcillus farcimi nis* | *Lactob αcillus crispat us* | *Lactob αcillus lactis* | *Lactob αcillus casei.* |
|---|---|---|---|---|---|---|---|---|
| The blank con trol | 0.838 | 1.304 | 0.245 | 1.010 | 0.678 | 0.677 | 0.716 | 0.627 |
| Example 1 | 0.963* | 1.646* | 0.373** | 1.482** | 0.858* | 0.879* | 1.001** | 0.775* |
| Example 2 | 0.930* | 1.577* | 0.337** | 1.424** | 0.811* | 0.853* | 0.952** | 0.742* |
| Example 3 | 0.892* | 1.494* | 0.329* | 1.392* | 0.788* | 0.836* | 0.932** | 0.729* |
| Example 4 | 0.927* | 1.521* | 0.341** | 1.413** | 0.805* | 0.850* | 0.948** | 0.737* |
| Example 5 | 0.906 | 1.509* | 0.334* | 1.405* | 0.794* | 0.847* | 0.940** | 0.732* |
| Example 6 | 0.882 | 1.476* | 0.317* | 1.361* | 0.769 | 0.811* | 0.919* | 0.721 |
| Example 7 | 0.891 | 1.484* | 0.323* | 1.372* | 0.775* | 0.819* | 0.927* | 0.725* |
| Example 8 | 0.876 | 1.441* | 0.303* | 1.357* | 0.757 | 0.808* | 0.875* | 0.713 |
| Example 9 | 0.933* | 1.584* | 0.360** | 1.433** | 0.826* | 0.857* | 0.957** | 0.747* |
| Example 10 | 0.951* | 1.610* | 0.354* | 1.457** | 0.841* | 0.871* | 0.981** | 0.769* |
| Example 11 | 0.941* | 1.592* | 0.365** | 1.441** | 0.832* | 0.868* | 0.966** | 0.751* |
| Comparative E xample 1 | 0.838 | 1.364 | 0.264 | 1.279 | 0.682 | 0.741 | 0.802 | 0.651 |
| Comparative E xample 2 | 0.842 | 1.396 | 0.276 | 1.294 | 0.691 | 0.773 | 0.825 | 0.674 |
| Comparative E xample 3 | 0.856 | 1.407 | 0.291 | 1.310 | 0.712 | 0.781 | 0.838 | 0.695 |
| Comparative E xample 4 | 0.831 | 1.363 | 0.257 | 1.274 | 0.675 | 0.747 | 0.798 | 0.642 |
| Comparative E xample 5 | 0.852 | 1.410 | 0.284 | 1.308 | 0.709 | 0.782 | 0.833 | 0.686 |
| Comparative E xample 6 | 0.830 | 1.321 | 0.251 | 1.182 | 0.670 | 0.691 | 0.742 | 0.631 |
| Comparative E xample 7 | 0.848 | 1.401 | 0.280 | 1.301 | 0.702 | 0.778 | 0.830 | 0.679 |

| | | | | | | | | |
|---|---|---|---|---|---|---|---|---|
| Note: Compared with the control group, *: P<0.05, significant; **: P<0.01, extremely significant. | | | | | | | | |

As can be seen from Table 4, the HA mixture has varying degrees of growth-promoting effects on the eight strains of probiotics. Taking the 12h data as an example, compared with the blank control group, the test samples may effectively increase the concentration of intestinal probiotics and number of viable bacteria. In particular, Example 1 has the best effect, followed by Examples 10, 11, 9, and 2, indicating that the hyaluronic acid composition has the function of promoting the growth of intestinal probiotics.

### Experiment Example 4 Experiment on relieving intestinal inflammation

Fluorescent zebrafish with development of 3dpf in which immune cells were specifically labeled were used as test animals, which were randomly divided into blank control group, model group and different sample groups. No drugs or substances to be tested were added to the blank control group. Trinitrobenzene sulfonic acid (TNBS) with a final concentration of 50 µg/mL was added to the model group and different sample groups at the same time, respectively. There were 10 larvae in each group, with 2 multiple holes in each group. The above zebrafish of each group were placed in a constant temperature incubator at 28.5°C and incubated continuously for 3 days in the dark. After the incubation, the larvae of each group were washed, and the larvae of the blank control group and the model group were placed in new culture water respectively, while the larvae of other groups were placed in culture water containing 100 µg/mL of different samples to be tested, and continued to be cultured at 28.5°C for 3 days. After the treatment, the number of immune cells showing red fluorescence in the zebrafish intestine were counted by using Imagepro-plus software, and the statistical analysis of the data was performed by using GraphPad software.

**Table 5 Effect of hyaluronic acid composition on gastrointestinal inflammation**

| Group | Dosage (µg/mL) | The number of immune cells |
|---|---|---|
| The control group | 100 | 19.9±0.936 |
| The model group | 100 | 28.9±1.441 ^{##} |
| Example1 | 100 | 20.3±0.615* |
| Example2 | 100 | 18.5±1.441** |
| Example3 | 100 | 18.2±1.013** |
| Example4 | 100 | 17.7±0.775** |
| Examples | 100 | 19.2±1.114* |
| Example6 | 100 | 18.9±1.174* |
| Example7 | 100 | 19.9±0.900* |
| Examples | 100 | 20.5±1.062* |
| Example9 | 100 | 19.9±0.632* |
| Example10 | 100 | 17.2±0.791** |
| Example11 | 100 | 19.2±0.466* |
| Comparative Example 1 | 100 | 24.7±0.800 |
| Comparative Example 2 | 100 | 24.2±1.012 |
| Comparative Example 3 | 100 | 23.5±1.061 |
| Comparative Example 4 | 100 | 22.7±1.067 |
| Comparative Example 5 | 100 | 22.3±1.078 |
| Comparative Example 6 | 100 | 25.5±0.614 |
| Comparative Example 7 | 100 | 23.4±0.723 |

| | | |
|---|---|---|
| Note: Compared with the control group, ##: P<0.01, extremely significant. Compared with the model group, *: P<0.05, significant; **: P<0.01, extremely significant. | | |

As can be seen from Table 5, compared with the blank control group, the number of immune cells in the intestinal tract of zebrafish in the model group increased significantly, with significant statistical significance; the result shows that when zebrafish are suffering from intestinal inflammation, the immune cells in the organism will migrate to the inflammatory site. When zebrafish were treated with different samples to be tested, compared with the model group, the number of immune cells in the intestinal tract was reduced, with extremely significant statistically significant; the result shows that the test products alleviated the inflammatory state in the intestinal tract of zebrafish in different degrees, therefore the number of immune cells migrating to the zebrafish intestinal tract also decreased.

In summary, the use of the composition described in the present application may significantly improve the number of immune cells in zebrafish with immune disorders, increase immune activity, promote intestinal peristalsis, increase the survival rate of various intestinal probiotics, improve intestinal bacteria group, and alleviate intestinal inflammation significantly, indicating that the composition described in the present application has significant effects on improving immunity and protecting the gastrointestinal tract, and may be added to food and health-care food as a functional ingredient, or be used in combination with drugs.

The above are only preferred embodiments of the present application and are not intended to limit the present application in other forms. Any skilled person familiar with the art may make changes or modifications to obtain equivalent examples with equivalent changes using the technical contents disclosed above. However, any simple modifications, equivalent changes, and modifications made to the above embodiments based on the technical essence of the present application without departing from the content of the technical solution of the present application still fall within the protection scope of the technical solution of the present application.

## Claims

1. A composition containing hyaluronic acids or salts thereof, which comprises hyaluronic acids or salts thereof with three different molecular weights, wherein:
the molecular weight of the hyaluronic acid or a salt thereof with a first molecular weight is between10 kDa and 200 kDa, preferably between 10 kDa and 100 kDa, further preferably between 30 kDa and 50 kDa;
the molecular weight of the hyaluronic acid or a salt thereof with a second molecular weight is between 200 kDa and 1000 kDa, preferably between 200 kDa and 600 kDa,
further preferably between 200 kDa and 400 kDa; and
the molecular weight of the hyaluronic acid or a salt thereof with a third molecular weight is between 1000 kDa and 1500 kDa, preferably between 1300 kDa and 1500 kDa.

2. The composition according to claim 1, wherein based on a total part of 10 parts by weight after mixing the three different hyaluronic acids,
the hyaluronic acid or a salt thereof with the first molecular weight is 1-4 parts, preferably 2-4 parts;
the hyaluronic acid or a salt thereof with the second molecular weight is 3-6 parts, preferably 4-6 parts; and
the hyaluronic acid or a salt thereof with the third molecular weight is 1-3 parts.

3. The composition according to any one of claims 1-2, wherein the hyaluronic acid or a salt thereof is one or two or more selected from the group consisting of hyaluronic acid, sodium hyaluronate, potassium hyaluronate, calcium hyaluronate and zinc hyaluronate, preferably sodium hyaluronate or potassium hyaluronate.

4. Use of the composition according to any one of claims 1-3 in the fields of food, health-care product or pharmaceutical.

5. A food composition, comprising the composition of any one of claims 1-3.

6. The food composition according to claim 5, wherein the food composition is used to improve organism immunity, relieve intestinal inflammation, increase intestinal peristalsis and/or regulate intestinal flora.

7. A health-care product composition comprising the composition according to any one of claims 1-3.

8. The health-care product composition according to claim 7, wherein the health-care product composition is used to improve organism immunity, relieve intestinal inflammation, increase intestinal peristalsis and/or regulate intestinal flora.

9. A pharmaceutical composition comprising the composition according to any one of claims 1-3.

10. The pharmaceutical composition according to claim 9, wherein the pharmaceutical composition is used to improve organism immunity, relieve intestinal inflammation, increase intestinal peristalsis and/or regulate intestinal flora.

11. A method for improving organism immunity, relieving intestinal inflammation, increasing intestinal peristalsis and/or regulating intestinal flora, which comprises administering the composition of any one of claims 1-3 to a subject.

12. Use of the composition according to any one of claims 1-3 for improving organism immunity, relieving intestinal inflammation, increasing intestinal peristalsis and/or regulating intestinal flora.

13. Use of the composition according to any one of claims 1-3 in the preparation of a medicament for improving organism immunity, relieving intestinal inflammation, increasing intestinal peristalsis and/or regulating intestinal flora.
